# EUROPEAN PATENT SPECIFICATION

(11) **EP 2 865 357 B1**
(45) Date of publication and mention of the grant of the patent: **13.02.2019**
(21) Application number: 14196715.8
(22) Date of filing: 09.12.2011
(51) Int. Cl.: A61F 2/58

(54) **Mechanical prosthetic finger device**
Mechanische prothetische Fingervorrichtung
Dispositif de prothèse mécanique de doigt

(30) Priority: 14.07.2011 US 201113183005
(43) Date of publication of application: 29.04.2015
(62) Divisional of application: 11192802.4
(73) Proprietor: MacDuff, Charles Colin, Olympia, WA 98501 (US)
(72) Inventor: MacDuff, Charles Colin, Olympia, WA 98501 (US)
(74) Representative: Hocking, Adrian Niall

(56) References cited:
- US-A- 319 776
- US-A- 2 867 819
- US-A1- 2005 043 822

## Description

### FIELD OF THE INVENTION

The present invention relates generally to a prosthetic device. More specifically, the present invention is a prosthetic device designed for full finger, partial finger, or finger tip amputees.

### BACKGROUND OF THE INVENTION

If a person loses a finger, a finger segment, or a finger tip, the result is impaired performance of the hand. Having an amputated finger inhibits an amputee from performing some of the most basic tasks. For example, with a lost finger or finger tip, the task of typing on a computer or simply dialing on a phone becomes significantly difficult. These types of tasks require actions with precision that only fingers are able to offer. Not only do fingers allow people to perform precise actions, but fingers also provide people with a increased ability to handle items. While holding an item in one hand, the weight of the item is dispersed through all of user's fingers. By simply varying the force used by each fingers on the holder's hands, the holder is able to manipulate the item in a myriad of ways. However, if the holder is missing a single finger, the amount of precision for the manipulation and the number of ways the holder can manipulate the item is decreased. US2005/043822 describes a mechanical prosthetic finger device to assist a person missing a single finger. The present invention is a device that acts as a prosthetic substitute of the lost portion of a finger. The present invention is designed to bend and naturally mimic a real finger. Additionally, the present invention comprises a metal thread looped about the tip of the finger to allow the users to interact with a capacitive type of touch screen.

### BRIEF DESCRIPTION OF THE DRAWINGS

FIG. 1 is a perspective view of the present invention.
FIG. 2 is a view of the present invention without the middle phalange showing the connection of the extended wishbone hinge to the pair of proximal pulling hinges.
FIG. 3 is an exploded view of the present invention.
FIG. 4 is a cross sectional view of the present invention showing the articulation cable and the touch screen mechanism.

### DETAIL DESCRIPTIONS OF THE INVENTION

All illustrations of the drawings are for the purpose of describing selected versions of the present invention and are not intended to limit the scope of the present invention.

The present invention is a prosthetic finger that can be fitted for a user with an amputated finger, finger tip, or finger segment. The prosthetic finger is a mechanical finger that is able to mimic the motions and functionalities of a real finger. The mechanical prosthetic finger comprises of three major components including a distal phalange **1,** a middle phalange **2,** and a proximal phalange ring **3.** A plurality of rods **8** and a series of hinges are used to secure the distal phalange **1,** the middle phalange **2,** and the proximal phalange ring **3** together. The distal phalange **1** is the tip segment of the prosthetic finger. The middle phalange **2** is the middle segment of the prosthetic finger. The proximal phalange ring **3** is the base of the prosthetic finger that anchors the entire prosthetic finger to the user's residual finger. As the level of amputation differs among each user, the present invention can be modified to be custom fit for each user. For example, users who have an amputated finger tip will be custom fitted with a prosthetic finger, where the middle phalange **2** and the proximal phalange ring **3** are frames that fit and mount to the user's residual finger. To provide the prosthetic finger with grip and a softer touch, the present invention additionally comprises a distal pad platform **4,** a distal pad **5,** a middle pad platform **6,** and a middle pad **7.** The distal pad **5** and the middle pad **7** are made from a soft texture that mimics the texture of a real finger. In the preferred embodiment of the present invention, to additionally contribute to the realistic aspect of the prosthetic finger, the present invention further comprises of an articulation cable **9** and a touch screen mechanism **10.** The articulation cable **9** further provides the prosthetic finger with realistic curling motions. The touch screen mechanism **10** allows the user to use the prosthetic finger to operate touch screens. Although some touch screens, such as resistive touch screens, only require pressure for sensing the touch, other touch screens uses the body's natural current to sense touch. These touch screens that require the user's natural body current are called capacitive touch screens. The touch screen mechanism **10** allows the user to conduct their own body current and direct it towards the tip of the prosthetic finger.

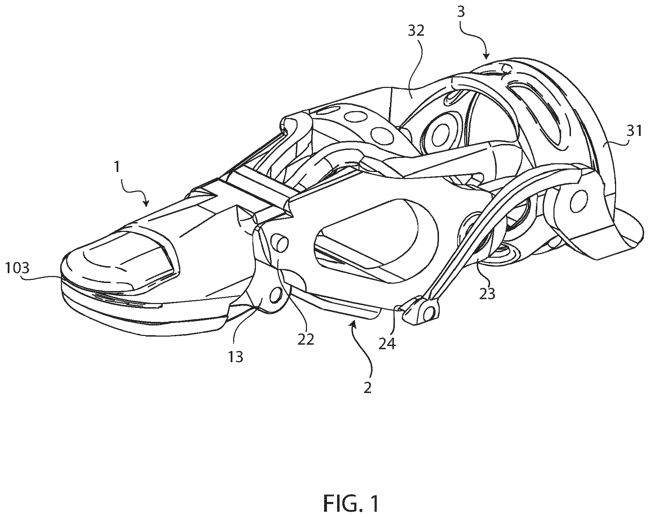

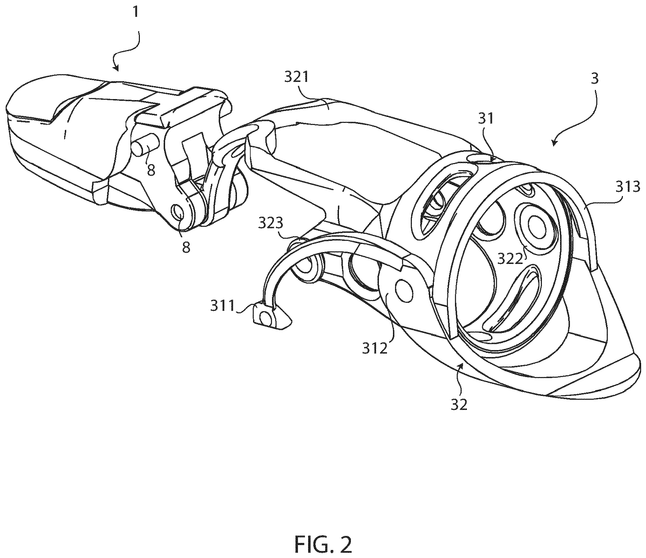

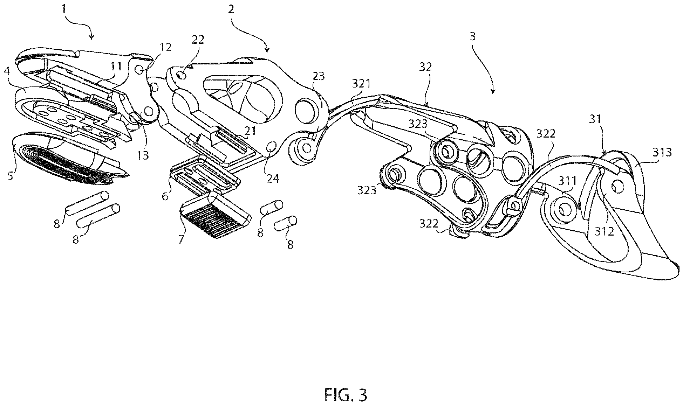

In reference to FIG. **1-3****,** the distal phalange **1** comprises a distal platform fastener **11,** a middle phalange joint channel **12,** and a pair of proximal pulling hinge. The distal pad **5** and the distal pad platform **4** are secured to the distal phalange **1.** The distal pad **5** is engaged and adhered to the distal pad platform **4** by a RTV silicone adhesive. The use of such an adhesive is important when using a silicone material for the distal pad **5** due to its high temperature material. The distal pad **5** is made from a soft material, such as silicone, to mimic the flesh of a real finger pad. The distal pad **5** is attached to the distal phalange **1** by means of the distal pad platform **4.** The distal pad platform **4** is secured to the distal platform fastener **11** of the distal phalange **1.** In the preferred embodiment of the present invention, the distal platform fastener **11** is a distal platform latch and the distal pad platform **4** comprises of a corresponding latch hole. However, in other embodiments of the present invention, the distal platform fastener **11** can be simply be an adhesive. The distal platform fastener **11** is positioned on a lower distal surface of the distal phalange **1.** In comparison to a real finger, the positioning of the distal platform fastener **11** allows the distal pad **5** to be positioned where the finger pads of a real finger would be. The distal phalange **1,** the distal pad **5,** and the distal pad platform **4** combine together to be shaped like a real finger tip. On the rear end of the distal phalange **1** is the middle phalange joint channel **12.** The middle phalange joint channel **12** is a hole that laterally traverses through the distal phalange **1.** The middle phalange joint channel **12** provides a pivot point for the connection of the middle phalange **2.** The pair of proximal pulling hinges **13** is a pair of hinge channels that downwardly extends at an angle from the rear of the distal phalange **1.** The pair of proximal pulling hinges **13** are positioned adjacent to the middle phalange joint channel **12.** The pair of proximal pulling hinges **13** provides a pulling point for the proximal phalange ring **3** to pull on to mimic the curling motion of a real finger.

In reference to FIG. **1-3****,** the middle phalange **2** comprises a middle platform fastener **21,** a pair of distal joint hinges **22,** a pair of proximal joint hinges **23,** and a pair of spring hinge ports **24.** For a finger amputee with a missing finger tip, the middle phalange **2** is a frame that wraps around the intermediate phalange of the user's residual finger. The middle pad **7** and the middle pad platform **6** are secured to the middle phalange **2.** The middle pad **7** is engaged and adhered to the middle pad platform **6** by a RTV silicone adhesive. Similar to the distal pad **5,** the middle pad **7** is made from a soft material, such as silicone. The middle pad **7** is attached to the middle phalange **2** by means of the middle pad platform **6.** The middle pad platform **6** is secured to the middle platform fastener **21** of the middle phalange **2.** In the preferred embodiment of the present invention, similar to the distal platform fastener **11,** the middle platform fastener **21** is a middle platform latch and the middle pad platform **6** comprises of a corresponding latch hole. In other embodiments, the middle platform fastener **21** can be an adhesive. The middle platform fastener **21** is positioned on a lower middle surface of the middle phalange **2.** Similar to the distal phalange **1,** the positioning of the middle platform fastened allows the middle pad **7** to be positioned where the finger pads of the intermediate phalange of a real finger would be. The middle phalange **2,** the middle pad **7,** and the middle pad platform **6** combine together to be shaped like a real intermediate phalange. The pair of distal joint hinges **22** is forwardly extended from the middle phalange **2** in parallel relationship to each other. The pair of proximal joint hinges **23** is extended from the middle phalange **2** in an opposite direction of the pair of distal joint hinges **22.** As a result, the pair of distal joint hinges **22** and the pair of proximal joint hinges **23** are positioned on opposite ends of the middle phalange **2.** The middle phalange **2** is able to jointly connect the distal phalange **1** to the proximal phalange ring **3** together by means of the pair of distal joint hinges **22** and the pair of proximal joint hinges **23.**

In reference to FIG. **1-3****,** the proximal phalange ring **3** is a two part component comprising of a proximal phalange yoke **31** and a proximal phalange frame **32.** The proximal phalange frame **32** is the body of the proximal phalange ring **3** that anchors itself onto the user's finger. The proximal phalange yoke **31** is the brace of the proximal phalange ring **3** that provides support in the motion provided by the present invention. The proximal phalange yoke **31** further comprises, a pair of extending spring hinges **311,** a pair of frame joint hinges **312,** and a finger base brace **313.** The proximal phalange frame **32** comprises an extended wishbone hinge **321,** a pair of posterior yoke joint hinge, and a pair of anterior phalange joint hinge **323.** The finger base brace **313** is a circular frame that is the body of the proximal phalange yoke **31.** The finger base brace **313** is shaped to fit the base of the user's residual finger. The pair of frame joint hinges **312** is extended from the finger base brace **313.** The pair of extending spring hinges **311** is a flat spring hinge that extends from torn the pair of frame joint hinges **312.** The extended wishbone bone is shaped like a wishbone and is forwardly extending from the proximal phalange frame **32.** The pair of anterior phalange joint hinges **323** is extended from the proximal phalange frame **32** adjacent to the extended wishbone hinge **321.** The pair of posterior yoke joint holes **322** are holes that laterally traverse through the proximal phalange. The proximal phalange yoke **31** is jointly connected to the proximal phalange frame **32.** The pair of frame joint hinges **312** is aligned and engaged to the pair of posterior yoke joint holes **322.** The pair of frame join hinges is able to jointly connect to the pair of posterior yoke joint holes **322** by means of a yoke stud. The yoke stud is inwardly protruding from each of the frame joint hinges. The proximal phalange yoke **31** is then aligned and jointly secured to the pair of posterior yoke joint holes **322.**

In reference to FIG. **1-3****,** the distal phalange **1** is connected to the middle phalange **2.** The proximal phalange ring **3** is connected to the middle phalange **2** opposite of the distal phalange **1.** The plurality of rods **8** is traversed through the pair of distal joint hinges **22,** the middle phalange joint channel **12,** the pair of proximal joint hinges **23,** the pair of extending spring hinges **311,** the extended wishbone hinge **321,** and the pair of proximal pulling hinge for the assembly. The plurality of rods **8** consists of a first rod, a second rod, and a third rod. The pair of distal joint hinges **22** is aligned and secured to the middle phalange joint channel **12** by the first rod. The pair of spring hinge ports **24** is aligned and secured to the pair of extending spring hinges **311** by the second rod. The extended wishbone hinge **321** is aligned and secured to the pair of proximal pulling hinges **13** by the third rod. The extended wishbone is extended over and traversed through the middle phalange **2** for its connection to the pair of proximal pulling hinges **13.** Each of the anterior phalange joint hinges **323** comprises a middle stud. The middle stud is an outwardly protruding stud from each anterior phalange joint hinge **323.** The pair of anterior phalange joint hinges **323** is aligned and jointly secured to the pair of proximal joint hinges **23** by the middle stud. All of the joint connections described provides the prosthetic finger the ability to curl and move like a real finger.

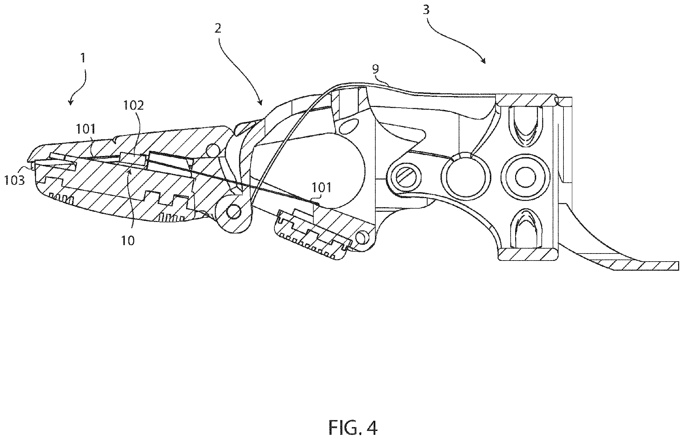

In reference to FIG. **4****,** the articulation cable **9** is connected to the proximal phalange frame **32** and the lower distal surface. The articulation cable **9** is traversed through the middle phalange **2** and contributes the life-like natural movements of the prosthetic finger. The touch screen mechanism **10** comprises a conductive thread **101,** and a conductive loop **103.** The conductive thread **101** consists of made out of a conductive material such as metal. The conductive loop **103** is the portion of the touch screen mechanism **10** that is used by the user to interact with the touch screen. The conductive loop **103** is made from a conductive material similar to the conductive thread **101.** The conductive loop **103** is connected directly to the conductive thread **101.** The conductive loop **103** is able to provide the user with the ability to interact with a touch screen at different angles. The distal phalange **1** having two holes and two channels is able to allow the conductive loop wrap around the tip of the distal phalange **1.** The two holes are positioned on a first distal corner and a second distal corner. Each of the holes are connected a respective channel. The conductive loop **103** is traversed through the two channels and connects to the second thread. The conductive loop **103** is left with an expose segment on the tip of the distal phalange **1** for interaction with a touch screen. To ensure that the touch screen mechanism **10** fully draws the user's natural body current, the conductive thread **101** can be connected to the finger base brace **313** to ensure contact with the user's flesh. In other embodiments of the present invention, the conductive thread **101** can be connected anywhere on the prosthetic finger as long as it makes contact with a user's flesh.

The present invention provides a comfortable and natural movement for a user with an amputated finger. The design can be individually customized for users with varying amounts of lose on their finger. To further provide better aesthetics, the present invention can be coated with colorings to match the user's skin. The ease of use is another advantage of the present invention. To use the present invention, the user can simply slide the prosthetic finger onto the appropriate finger like a ring. To curl and bend the prosthetic finger the used can utilize the natural movements of the residual finger that the device is being worn on. The finger segments will articulate using the same cognitive process that was previously utilized for their original finger. Each of the prosthetic fingers can be independently operated. This means the user will be able to perform the activities including full typing, playing a musical instrument, or anything that requires the full dexterity of a hand. The present invention is fully powered by the user's own body. Each components of the prosthetic finger is able to move simply based on the actions of the user's residual finger. The present invention is designed to offer strength in the lowest profile design. As a result, the present invention naturally conforms with the looks of the user's hand.

Medical benefits of the present invention include uses of the device that reduce swelling and increases circulation, supporting the adjacent finger joints. The present device can be made out of Titanium, Stainless Steel, Aluminum, Silicone, Carbon Fiber, Nylon, Plastic, Wood, Rubber, Gold, Silver, Tungsten, Flex Cable, neoprene or any suitable structural material that is non-irritating to human skin. However, in the preferred embodiment of the present invention, the device is made from the material Duraform EX polymer material.

It may be possible that portions of the prosthetic finger can be used for differing conditions of the user. The present invention can be accommodated for finger tips or full fingers. The extended wishbone hinge **321** can be removed so that the prosthetic finger can be used as joint brace. Additionally, using biocompatible materials, the present invention can be applied as an orthopedic implant. Depending on the condition of the user, the present invention can be surgically implanted into the user's fingers. The use of the surgical implantation of the present invention can be applied for users having injuries that have crushed their bones without the ability to heal and be repaired. As a result, the present invention is able to take the place of the user's original bones without the need for amputation.

## Claims

1. A mechanical prosthetic finger device comprising,
a distal phalange (1) providing a tip segment of a prosthetic finger;
a proximal phalange ring (3) having a proximal phalange frame (32) configured to concentrically receive a user's residual finger;
a middle phalange (2) configured to concentrically receive the user's residual finger, the middle phalange (2) having a pair of distal joint hinges (22), and a pair of proximal joint hinges (23), the middle phalange (2) providing a joint connection of the distal phalange (1) and the proximal phalange frame (32) by the distal joint hinges (22) to the distal phalange (1), and by proximal joint hinges (23) to the proximal phalange frame (32); and
**characterized by** a continuous articulation cable (9) traversing through the middle phalange (2), the articulation cable (9) having a distal end and a proximal end, the distal end being anchored to the distal phalange (1) and the proximal end being anchored to the proximal phalange frame (32);
wherein the middle phalange (2) together with the articulation cable (9) are configured to utilise articulation movements of the residual finger within the proximal phalange frame (32) to curl and bend the distal phalange (1).

2. A mechanical prosthetic finger device as claimed in claim 1, wherein the distal phalange (1) includes a distal pad platform (4) with a distal pad (5).

3. A mechanical prosthetic finger device as claimed in claim 1 or claim 2, wherein the middle phalange (2) includes a middle finger platform (6) with a middle pad (7).

4. A mechanical prosthetic finger device as claimed in any one of the preceding claims, wherein the distal phalange (1) has a middle phalange joint channel (12), and the distal joint hinges (22) connect to the middle phalange joint channel (12).

5. A mechanical prosthetic finger device as claimed in any one of the preceding claims, wherein the distal phalange (1) includes a pair of proximal pulling hinges (13).

6. A mechanical prosthetic finger device as claimed in any one of the preceding claims, wherein the proximal phalange frame (32) includes a wishbone hinge having a distal end that hingedly connects with the distal phalange (1).

7. A mechanical prosthetic finger device as claimed in any one of the preceding claims, wherein the middle phalange (2) includes a frame configured to wrap around an intermediate phalange of a user's residual finger.

8. A mechanical prosthetic finger device as claimed in any one of the preceding claims, wherein the distal phalange (1), the middle phalange (2), and the proximal phalange include a polymer material.

9. A mechanical prosthetic finger device as claimed in any one of the preceding claims, wherein the distal phalange (1), the middle phalange (2), and the proximal phalange include a rubber material.

10. A mechanical prosthetic finger device as claimed in any one of the preceding claims, wherein at least one of the distal phalange (1), the middle phalange (2), and the proximal phalange slidingly receive the user's residual finger.

11. A mechanical prosthetic finger device as claimed in claim 10, wherein the proximal phalange slidingly receives the user's residual finger of a full finger amputation.

12. A mechanical prosthetic finger device as claimed in claim 10, wherein the proximal phalange and the middle phalange (2) slidingly receive the user's residual finger of a partial finger amputation.

13. A mechanical prosthetic finger device as claimed in claim 10, wherein the proximal phalange, the middle phalange (2), and the distal phalange (1) slidingly receive the user's residual finger of a finger tip amputation.

14. A mechanical prosthetic finger device as claimed in any one of the preceding claims, wherein the distal phalange (1) includes a touch screen mechanism (10) to interact with a touch screen.

15. A mechanical prosthetic finger device as claimed in claim 14, wherein the touch screen mechanism (10) includes a conductive loop (103) having an exposed segment on a tip of the distal phalange, the conductive loop (103) drawing from the user's natural body current.

## Patentansprüche

1. Mechanische prothetische Fingervorrichtung, umfassend,
eine distale Phalanx (1), die ein Spitzensegment eines prothetischen Fingers bereitstellt;
einen proximalen Phalanxring (3) mit einem proximalen Phalanxrahmen (32), der konfiguriert ist, um den Restfinger eines Benutzers konzentrisch aufzunehmen;
eine mittlere Phalanx (2), die konfiguriert ist, um den Restfinger des Benutzers konzentrisch aufzunehmen, wobei die mittlere Phalanx (2) ein Paar distale Gelenkscharniere (22) und ein Paar proximale Gelenkscharniere (23) aufweist, wobei die mittlere Phalanx (2) eine Gelenkverbindung der distalen Phalanx (1) und des proximalen Phalanxrahmens (32) durch die distalen Gelenkscharniere (22) mit der distalen Phalanx (1) und durch proximale Gelenkscharniere (23) mit den proximalen Phalanxrahmen (32) bildet; und
**gekennzeichnet durch** ein kontinuierliches Artikulierungskabel (9), das durch die mittlere Phalanx (2) verläuft, wobei das Artikulierungskabel (9) ein distales Ende und ein proximales Ende aufweist, wobei das distale Ende an der distalen Phalanx (1) verankert ist und das proximale Ende am proximalen Phalanxrahmen (32) befestigt ist;
wobei die mittlere Phalanx (2) zusammen mit dem Artikulierungskabel (9) konfiguriert sind, um Artikulierungsbewegungen des Restfingers innerhalb des proximalen Phalanxrahmens (32) zu nutzen, um die distale Phalanx (1) zu krümmen und zu biegen.

2. Mechanische prothetische Fingervorrichtung nach Anspruch 1, wobei die distale Phalanx (1) eine distale Polsterplattform (4) mit einem distalen Polster (5) beinhaltet.

3. Mechanische prothetische Fingeranordnung nach Anspruch 1 oder Anspruch 2, wobei die mittlere Phalanx (2) eine Mittelfingerplattform (6) mit einem mittleren Polster (7) beinhaltet.

4. Mechanische prothetische Fingervorrichtung nach einem der vorhergehenden Ansprüche, wobei die distale Phalanx (1) einen mittleren Phalanx-Gelenk-Kanal (12) aufweist, und die distalen Gelenkscharniere (22) mit dem mittleren Phalanx-Gelenk-Kanal (12) verbunden sind.

5. Mechanische prothetische Fingervorrichtung nach einem der vorhergehenden Ansprüche, wobei der distale Fingerknochen (1) ein Paar proximaler Zuggelenke (13) beinhaltet.

6. Mechanische prothetische Fingeranordnung nach einem der vorhergehenden Ansprüche, wobei der proximale Phalanxrahmen (32) ein Querlenkerscharnier mit einem distalen Ende umfasst, das gelenkig mit der distalen Phalanx (1) verbunden ist.

7. Mechanische prothetische Fingervorrichtung nach einem der vorhergehenden Ansprüche, wobei die mittlere Phalanx (2) einen Rahmen beinhaltet, der konfiguriert ist, um eine mittlere Phalanx des Restfingers eines Benutzers zu umschließen.

8. Mechanische prothetische Fingervorrichtung nach einem der vorhergehenden Ansprüche, wobei die distale Phalanx (1), die mittlere Phalanx (2) und die proximale Phalanx ein Polymermaterial umfassen.

9. Mechanische prothetische Fingervorrichtung nach einem der vorhergehenden Ansprüche, wobei die distale Phalanx (1), die mittlere Phalanx (2) und die proximale Phalanx ein Gummimaterial umfassen.

10. Mechanische prothetische Finger nach einem der vorhergehenden Ansprüche, wobei mindestens eine der distalen Phalanx (1), der mittleren Phalanx (2) und der proximalen Phalanx gleitend den Restfinger des Benutzers aufnehmen.

11. Mechanische prothetische Fingervorrichtung nach Anspruch 10, wobei die proximale Phalanx den Restfinger des Benutzers nach einer vollständigen Fingeramputation gleitend aufnimmt.

12. Mechanische prothetische Fingervorrichtung nach Anspruch 10, wobei die proximale Phalanx und die mittlere Phalanx (2) den Restfinger des Benutzers nach einer teilweisen Fingeramputation gleitend aufnehmen.

13. Mechanische prothetische Fingeranordnung nach Anspruch 10, wobei die proximale Phalanx, die mittlere Phalanx (2) und die distale Phalanx (1) den Restfinger des Benutzers nach einer Fingerspitzenamputation verschiebbar aufnehmen.

14. Mechanische prothetische Fingervorrichtung nach einem der vorhergehenden Ansprüche, wobei die distale Phalanx (1) einen Berührungsbildschirmmechanismus (10) zum Interagieren mit einem Berührungsbildschirm umfasst.

15. Mechanische prothetische Fingervorrichtung nach Anspruch 14, wobei der Berührungsbildschirmmechanismus (10) eine leitfähige Schleife (103) mit einem freiliegenden Segment an einer Spitze der distalen Phalanx umfasst, wobei die leitfähige Schleife (103) aus dem natürlichen Körperstrom des Benutzers entnommen wird.

## Revendications

1. Dispositif de prothèse mécanique de doigt comprenant :
une phalange distale (1) procurant un segment d'extrémité d'une prothèse de doigt ;
une bague proximale de phalange (3) comportant une armature de phalange proximale (32) conçue pour recevoir concentriquement un doigt résiduel d'utilisateur ;
une phalange intermédiaire (2), conçue pour recevoir concentriquement le doigt résiduel de l'utilisateur, la phalange intermédiaire (2) comportant une paire d'articulations distales de jointure (22) et une paire d'articulations de jointure proximale (23), la phalange intermédiaire (2) procurant une liaison de jointure de la phalange distale (1) et de l'armature de phalange proximale (32) par les articulations de jointure distale (22) à la phalange distale (1), et par des articulations de jointure proximale (23) à l'armature de phalange proximale (32) ; et
**caractérisé par** un câble continu d'articulation (9) traversant la phalange intermédiaire (2), le câble d'articulation (9) comportant une extrémité distale et une extrémité proximale, l'extrémité distale étant ancrée à la phalange distale (1) et l'extrémité proximale étant ancrée à l'armature de phalange proximale (32) ;
la phalange intermédiaire (2), conjointement avec le câble d'articulation (9) étant conçue pour utiliser les mouvements d'articulation du doigt résiduel à l'intérieur de l'armature de phalange proximale (32) pour courber et pour incurver la phalange distale (1).

2. Dispositif de prothèse mécanique de doigt selon la revendication 1, dans lequel la phalange distale (1) contient une plateforme à tampon distal (4) dotée d'un tampon distal (5).

3. Dispositif de prothèse mécanique de doigt selon la revendication 1 ou la revendication 2, dans lequel la phalange intermédiaire (2) contient une plateforme intermédiaire de doigt (6) dotée d'un tampon intermédiaire (7).

4. Dispositif de prothèse mécanique de doigt selon l'une quelconque des revendications précédentes, dans lequel la phalange distale (1) comporte un canal de jointure de phalange intermédiaire (12) et où les articulations de jointure distale (22) se lient canal de jointure de phalange intermédiaire (12).

5. Dispositif de prothèse mécanique de doigt selon l'une quelconque des revendications précédentes, dans lequel la phalange distale (1) contient une paire d'articulations proximales de traction (13).

6. Dispositif de prothèse mécanique de doigt selon l'une quelconque des revendications précédentes, dans lequel l'armature de phalange proximale (32) contient une articulation à bréchet comportant une extrémité distale qui se lie de manière articulée à la phalange distale (1).

7. Dispositif de prothèse mécanique de doigt selon l'une quelconque des revendications précédentes, dans lequel la phalange médiane (2) contient une armature conçue pour s'envelopper autour d'une phalange intermédiaire d'un doigt résiduel d'utilisateur.

8. Dispositif de prothèse mécanique de doigt selon l'une quelconque des revendications précédentes, dans lequel l'une quelconque de la phalange distale (1), de la phalange intermédiaire (2) et de la phalange proximale contiennent un matériau polymère.

9. Dispositif de prothèse mécanique de doigt selon l'une quelconque des revendications précédentes, dans lequel l'une quelconque de la phalange distale (1), de la phalange intermédiaire (2) et de la phalange proximale contiennent un matériau à base de caoutchouc.

10. Dispositif de prothèse mécanique de doigt selon l'une quelconque des revendications précédentes, tel que le doigt résiduel de l'utilisateur peut se glisser dans l'une quelconque de la phalange distale (1), de la phalange intermédiaire (2) et de la phalange proximale.

11. Dispositif de prothèse mécanique de doigt selon la revendication 10, tel que le doigt résiduel de l'utilisateur peut, à la suite d'une amputation totale de doigt, se glisser dans la phalange proximale.

12. Dispositif de prothèse mécanique de doigt selon la revendication 10, tel que le doigt résiduel de l'utilisateur peut, à la suite d'une amputation partielle de doigt, se glisser dans la phalange proximale et dans la phalange intermédiaire (2).

13. Dispositif de prothèse mécanique de doigt selon la revendication 10, tel que le doigt résiduel de l'utilisateur peut, à la suite d'une amputation de bout de doigt, se glisser dans la phalange proximale, dans la phalange intermédiaire (2) et dans la phalange distale (1).

14. Dispositif de prothèse mécanique de doigt selon l'une quelconque des revendications précédentes, dans lequel la phalange distale (1) comprend un mécanisme à écran tactile (10) permettant d'interagir avec un écran tactile.

15. Dispositif de prothèse mécanique de doigt selon la revendication 14, dans lequel le mécanisme à écran tactile (10) comprend une boucle conductrice (103) comportant un segment exposé sur un bout de la phalange distale, la boucle conductrice (103) faisant circuler du courant électrique naturel du corps de l'utilisateur.
